(19) Europäisches Patentamt
European Patent Office
Office européen des brevets

(11) **EP 4 480 400 A1**

(12) **EUROPEAN PATENT APPLICATION**

(43) Date of publication:
**25.12.2024   Bulletin 2024/52**

(21) Application number: **23185449.8**

(22) Date of filing: **14.07.2023**

(51) International Patent Classification (IPC):
*A61B 5/021* (2006.01)     *A61B 5/029* (2006.01)
*A61B 5/00* (2006.01)     *G16H 50/20* (2018.01)
*G06N 3/08* (2023.01)

(52) Cooperative Patent Classification (CPC):
**A61B 5/7267; A61B 5/021; A61B 5/029;**
**G06N 3/0464; G06N 3/09; G16H 50/20**

(84) Designated Contracting States:
**AL AT BE BG CH CY CZ DE DK EE ES FI FR GB**
**GR HR HU IE IS IT LI LT LU LV MC ME MK MT NL**
**NO PL PT RO RS SE SI SK SM TR**
Designated Extension States:
**BA**
Designated Validation States:
**KH MA MD TN**

(30) Priority:  **23.06.2023   US 202363522732 P**

(71) Applicant: **Koninklijke Philips N.V.**
**5656 AG Eindhoven (NL)**

(72) Inventor: **NATARAJAN, Annamalai**
**Eindhoven (NL)**

(74) Representative: **Philips Intellectual Property &**
**Standards**
**High Tech Campus 52**
**5656 AG Eindhoven (NL)**

(54) **SYSTEM AND METHOD FOR ESTIMATING CARDIAC OUTPUT**

(57)     A system 100 and method are provided for estimating cardiac output of a patient from patient monitoring data 55. The patient monitoring data 55 may comprise a sequence of arterial blood pressure waveforms 50. A trained machine learning model 40 may be provided which may have been previously trained to estimate the cardiac output from a sequence of arterial blood pressure waveforms. The trained machine learning model may be used for this purpose by selecting an input time segment from the patient monitoring data 55 and using the input time segment as input to the trained machine learning model to obtain an estimated cardiac output as output. Advantageously, by training and applying a machine learning model directly on arterial blood pressure waveforms, instead of on features extracted from the arterial blood pressure waveforms, it may be avoided that inaccuracies and errors in the feature extraction, and in the segmentation of the waveform which typically precedes such feature extraction, propagate into the estimate of the cardiac output.

Fig. 1

**EP 4 480 400 A1**

**Description**

FIELD OF THE INVENTION

The invention further relates to an estimation system and computer-implemented estimation method for estimating cardiac output of a patient from patient monitoring data. The invention further relates to a training system and computer-implemented training method for training a machine learning model to estimate the cardiac output of the patient from the patient monitoring data. The invention further relates to a patient monitor comprising the estimation system, and to a computer-readable medium comprising instructions to cause a processor system to perform any of the computer-implemented methods.

BACKGROUND OF THE INVENTION

It is known to monitor one or more physiological parameters of a patient at regular and consecutive time instances. For example, in an intensive care unit (ICU), a patient's heart rate (HR), respiration rate (RR), core body temperature (CBT), oxygen saturation (SpO2) and blood pressure (BP) may be measured and visualized to clinical staff. Such monitoring of physiological parameters may also be referred to as 'patient monitoring'. Typically, in patient monitoring, measured values are compared to static or dynamic thresholds so detect abnormalities in the physiological parameters, and if such abnormalities are detected, an alarm may be triggered, e.g., to attract the attention of clinical staff.

An important physiological parameter is cardiac output (CO). For example, the cardiac output may serve as a clinical marker in an ICU. Cardiac output is also frequently used as an input parameter when assessing hemodynamic instability, fluid balance, fluid resuscitation, shock type classification, etc. It is known to determine the cardiac output of a patient using different techniques which may range from fully invasive to minimally invasive to non-invasive techniques. Each measurement technique has its own advantages and disadvantages and the selection of a measurement technique typically involves determining which trade-offs between accuracy, possible complications and changes in clinical workflow is acceptable. Disadvantageously, the most accurate techniques for measuring cardiac output may only used on the sickest patient populations, which may result in clinical bias seeping into the mere availability of measurements of cardiac output.

Arterial blood pressure (ABP) monitoring is part of standard clinical practice in ICU's. Such arterial blood pressure monitoring may be carried out using arterial lines (also referred to as art-line or A-Line or intra-arterial line), with an arterial line also enabling clinical staff to draw blood samples and administer medications. By continuous arterial blood pressure monitoring, the ABP waveforms of a patient may be measured. ABP waveforms normally comprise a systolic phase, a dicrotic notch and a diastolic phase, and the morphology of a ABP waveform typically carries relevant diagnostic information.

Techniques have been developed to estimate cardiac output from ABP waveforms. For example, in the approach described in reference [1], the ABP waveforms are segmented, features of interest are extracted from the segmented ABP waveforms, and the features are then used in mathematical models of human physiology to estimate cardiac output, for example using the Liljestrand and Zander formula. A disadvantage of the approach of [1] is that inaccuracies and errors in the segmentation of the ABP waveform and in the feature extraction may propagate into the estimate of cardiac outputs, and may thus render the estimated cardiac output noisy and unusable. Indeed, an error of one standard deviation of 0.8 litres/minute over 120 subjects has been reported, which is roughly twice that of the error from the invasive thermodilution technique that is considered the gold standard [1].

**References**

[1] Sun, J. X., Reisner, A. T., Saeed, M., & Mark, R. G. (2005, September). Estimating cardiac output from arterial blood pressure waveforms: a critical evaluation using the MIMIC II database. In Computers in Cardiology, 2005 (pp. 295-298). IEEE.

SUMMARY OF THE INVENTION

It would be desirable to estimate the cardiac output from ABP waveforms in a manner which addresses at least one of the disadvantages of the approach of [1].

In a first aspect of the invention, a training system is provided to train a machine learning model to estimate cardiac output of a patient from patient monitoring data. The training system comprises:

an input interface to access training data, wherein the training data comprises a set of training data instances, wherein each training data instance comprises a time-series of measurements of arterial blood pressure of a patient and a quantification of cardiac output of the patient, wherein the time-series of measurements is obtained by arterial line and comprises a sequence of arterial blood pressure waveforms;

a processor subsystem configured to train the machine learning model on the training data by, for a respective training data instance:

- select an input time segment from the time-series of measurements, wherein the input time segment is selected to at least in part temporally precede a time instance for which the quantification of the cardiac output is available;
- perform a training pass by using the input time segment as input and the quantification of the cardiac output as prediction target.

In a further aspect of the invention, a computer-implemented method is provided for training a machine learning model to estimate cardiac output of a patient from patient monitoring data. The method comprises:

accessing training data, wherein the training data comprises a set of training data instances, wherein each training data instance comprises a time-series of measurements of arterial blood pressure of a patient and a quantification of cardiac output of the patient, wherein the time-series of measurements is obtained by arterial line and comprises a sequence of arterial blood pressure waveforms;

training the machine learning model on the training data by, for a respective training data instance:

- selecting an input time segment from the time-series of measurements, wherein the input time segment is selected to at least in part temporally precede a time instance for which the quantification of the cardiac output is available;
- performing a training pass by using the input time segment as input and the quantification of the cardiac output as prediction target.

In a further aspect of the invention, an estimation system is provided to estimate cardiac output of a patient from patient monitoring data. The estimation system comprises:

an input interface to access the patient monitoring data, wherein the patient monitoring data comprises a time-series of measurements of arterial blood pressure of the patient, wherein the time-series of measurements is obtained by arterial line and comprises a sequence of arterial blood pressure waveforms;

an output interface;

a processor subsystem configured to:

- provide a trained machine learning model which is trained to estimate cardiac output from a time segment of measurements of the arterial blood pressure;
- select an input time segment from the patient monitoring data;
- use the input time segment as input to the trained machine learning model to obtain an estimated cardiac output as output; and
- output the estimated cardiac output via the output interface.

In a further aspect of the invention, a computer-implemented method is provided for estimating cardiac output of a patient from patient monitoring data. The method comprises:

accessing the patient monitoring data, wherein the patient monitoring data comprises a time-series of measurements of arterial blood pressure of the patient, wherein the time-series of measurements is obtained by arterial line and comprises a sequence of arterial blood pressure waveforms;

providing a trained machine learning model which is trained to estimate cardiac output from a time segment of measurements of the arterial blood pressure;

selecting an input time segment from the patient monitoring data; and

using the input time segment as input to the trained machine learning model to obtain an estimated cardiac output as output.

In a further aspect of the invention, a transitory or non-transitory computer-readable medium is provided, the computer-readable medium comprising data representing a computer program comprising instructions for causing a processor system to perform a computer-implemented method as described in this specification.

The above aspects of the invention involve training a machine learning model to estimate cardiac output from patient monitoring data and subsequently using the trained machine learning model in clinical practice for said purpose. To train the machine learning model, training data instances may be accessed which each comprise a time-series of measurements of arterial blood pressure of a patient and a quantification of cardiac output of the patient. The time-series of measurements may be arterial line measurements and may measure and thereby represent the ABP waveforms of the patient within a certain time frame, for example within a time frame of 1 minute or longer. Accordingly, each training instance may comprise a number of ABP waveforms, and additionally, the aforementioned quantification of cardiac output. Such a quantification may be obtained using known techniques, and in particular, using techniques which do not directly rely on the ABP waveforms. For example, the cardiac output may be determined by thermodilution or using the Fick principle. Typically, different training data instances pertain to different patients but several of the training data instances may also pertain to a same patient.

The training of the machine learning model may involve selecting an input time segment from the measurement data of a patient, that is, from the time-series of measurements of the patient. Typically, the input time segment is selected as a time window within the measurement data, but may in some cases also include all of the measurement data of the patient. The input time segment may be specifically selected to entirely temporally precede a time instance for which the quantification of the cardiac output is available. This may be explained as follows. The quantification of the cardiac output may pertain to a particular moment in time, e.g., when it was measured or when the physiological parameters on which the cardiac output is based were measured or when a last one of the physiological parameters was measured. The input time segment may be selected to contain multiple ABP waveforms before this particular moment in time, and in some cases, the input time segment may be selected to entirely lie before this moment in time.

The selected time segment may then be used as input to the machine learning model in a training pass, and in a specific example, in a so-called forward pass of the training. During this training pass, the quantification of the cardiac output may be used as a prediction target. For example, the quantification of the cardiac output may be used in a loss function so as to adjust weights or other parameters of the machine learning model in a backward pass of the training. This way, the machine learning model may be trained to predict the cardiac output from time segments of measurements of the arterial blood pressure of a patient. When deployed in clinical practice, measurements of the arterial blood pressure of a patient may be obtained via arterial line and accessed in real-time or near-real time by the estimation system and method to estimate the cardiac output of the patient. This cardiac output may then be estimated periodically, e.g., every n-th second or every m-th minute, and visualized to clinical staff, for example as a numeric value or in any other way.

The above measures may have the effect that the cardiac output may be directly estimated from ABP waveforms obtained from arterial lines. Such arterial lines may already be used in many clinical settings, e.g., in an ICU, to monitor the arterial blood pressure but also to draw blood samples and administer medications. By using the ABP waveforms, no additional invasive measures may have to be taken in relation to the patient. In particular, it may not be needed to take any invasive measures for the purpose of estimating the cardiac output since ABP measurement data may already be available in many clinical settings. In addition, by training a machine learning model directly on the ABP waveforms, instead of on features extracted from the ABP waveforms, it may be avoided that inaccuracies and errors in the feature extraction, and in the segmentation of the ABP waveform which typically precedes such feature extraction, propagate into the estimate of the cardiac output. This way, a more accurate and less noisy estimate of the cardiac output of a patient may be obtained. Another advantage may be that due to the ubiquitousness of arterial line measurements in clinical practice, the estimation of cardiac output may be deployed to a more diverse patient population, and not only the sickest patient populations, which may mitigate the problem of clinical bias seeping into the mere availability of measurements of cardiac output. This in turn may enable unbiased or less biased statistical analysis of the measurements of cardiac output, e.g., for clinical research purposes.

CN113057617A describes a non-invasive monitoring system for cardiac output which uses a trained LSTM network. However, the LSTM network is applied to features which are extracted from segmented ABP waveforms. This makes the prediction by the LSTM to be susceptible to the same type of problems as reference [1].

The following optional aspects may, unless otherwise noted or precluded for technical reasons, apply equally to the training and the subsequent use of the machine learning model, and apply equally to a respective system and corresponding method.

Optionally, the machine learning model is or comprises a temporal convolutional network. The inventor(s) has (have) recognized that a temporal convolutional networks (TCN) has properties which make the TCN very suitable for the estimation of cardiac output from ABP waveforms. For example, the convolutional nature of a TCN allows the TCN to base its prediction (i.e., its estimate of the cardiac output) on the shape of ABP waveforms, which may eliminate the necessity to segment the ABP waveforms and extract features from the segmented ABP waveforms. Yet another example is that the temporal natural of a TCN allows the TCN to base its prediction on the temporal characteristics of a series of ABP waveforms, e.g., on their temporal distance, their repetition, etc. Yet another example is that a TCN may use input sequences of varying lengths, which may be especially desirable during training as training data instances may comprise different numbers of ABP waveforms. Another example is that a TCN may capture long range dependencies which may allow the prediction to be based also on the distant past next to the recent past.

Optionally, the input time segment is selected to have a time length between 30 seconds and 10 minutes. It has been found that, given the dynamic nature of cardiac output, a history of ABP waveforms having a time length selected between 30 seconds and 10 minutes allows the cardiac output to be sufficiently accurately estimated.

Optionally, the input time segment is selected within or as a time window which ends at or is temporally adjacent to the time instance for which the quantification of the cardiac output is available, for example within a ten-minute time window. Both the distant past and the recent past may be relevant in the prediction of cardiac output. To ensure that the recent past is available, in particular during the training, the ABP waveforms which may be selected as input for the machine learning model may be required to lie at least in part within the recent past, for example within the aforementioned ten-minute time window which precedes the time instance for which the quantification of the cardiac output is available.

Optionally, in the training of the machine learning model, quantifications of cardiac output are used, or exclusively used, which are obtained by measurement using thermodilution. It was found that the use of training data instances which between themselves use different quantifications of cardiac output, such as thermodilution and the Fick principle, may reduce the accuracy by which the machine learning model estimates the cardiac output. In other words, the mixing of different types of quantifications of cardiac output in the training data was found to be disadvantageous. To address this problem, training data may be used of which the cardiac output is exclusively obtained by a same type of technique, being preferably thermodilution. This way, the machine learning model may be trained to provide a more accurate estimate of the cardiac output.

Optionally, in the training of the machine learning model, the machine learning model exclusively uses the input time segment as input. In other words, only raw arterial blood pressure waveforms may be used in the training, and additional hand-crafted features (e.g., characterizing the systolic/diastolic phases) may be expressly omitted. By forgoing additional feature extraction, it may be avoided that inaccuracies and errors in the feature extraction, and/or in the segmentation of the ABP waveform which typically precedes such feature extraction, propagate into the estimate of the cardiac output.

Optionally, measurements within the input time segment are normalized before using the input time segment in the training pass to fit within a predetermined range, for example between 0 and 1 or between -1 and 1.

Optionally, when using the machine learning model to estimate the cardiac output of a patient, e.g., in clinical practice, the patient monitoring data is accessed as a real-time or near-real time stream of measurements, and periodic estimates of the cardiac output are provided by periodically selecting an input time segment which includes and extends into the past from a most recent measurement.

Optionally, a periodicity of the estimates of the cardiac output and a length of the input time segment are selected such that consecutive input time segments overlap in time. By using overlapping input time segments, the frequency of cardiac output estimates may be increased compared to the use of non-overlapping input time segments. This may allow clinical staff to better monitor the patient's cardiac output.

In a further aspect of the invention, a patient monitor is provided comprising the estimation system as described in this specification. The estimation system may thus be a subsystem of the patient monitor, and any input and output interfaces may be internal interfaces of the subsystem and patient monitor.

It will be appreciated by those skilled in the art that two or more of the above-mentioned embodiments, implementations, and/or optional aspects of the invention may be combined in any way deemed useful.

Modifications and variations of any system, any computer-implemented method and/or the computer program product, which correspond to the described modifications and variations of another one of said entities, can be carried out by a person skilled in the art on the basis of the present description.

BRIEF DESCRIPTION OF THE DRAWINGS

These and other aspects of the invention will be apparent from and elucidated further with reference to the embodiments described by way of example in the following description and with reference to the accompanying drawings, in which:

Fig. 1 shows a training system configured to use a machine learning model to estimate cardiac output of a patient from arterial blood pressure measurement data;

Fig. 2 shows an estimation system configured to train the machine learning model using training data which comprises a plurality of training data instances;

Fig. 3 shows a time-sequence of arterial blood pressure waveforms and a time instance for which a quantification of cardiac output is available;

Fig. 4 shows a method for training the machine learning model;

Fig. 5 shows a method for using the trained machine learning model to estimate the cardiac output of a patient from arterial blood pressure measurement data; and

Fig. 6 shows a non-transitory computer-readable medium comprising data.

It should be noted that the figures are purely diagrammatic and not drawn to scale. In the figures, elements which correspond to elements already described may have the same reference numerals.

**List of reference numbers**

The following list of reference numbers is provided for facilitating the interpretation of the drawings and shall not be construed as limiting the claims.

| | |
|---|---|
| 20, 22 | data storage |
| 30 | training data |
| 40 | machine learning model |
| 50 | arterial blood pressure waveforms |
| 55 | patient monitoring data |
| 60 | display |
| 62 | display data |
| 80 | user input device |
| 82 | user input data |

| | |
|---|---|
| 100 | system for estimating cardiac output |
| 110 | data storage interface |
| 120 | input interface |
| 140 | processor subsystem |
| 142-148 | data communication |
| 160 | memory |
| 180 | user interface subsystem |
| 182 | display output interface |
| 184 | user input interface |

| | |
|---|---|
| 200 | system for training machine learning model |
| 220 | data storage interface |
| 240 | processor subsystem |
| 242-246 | data communication |
| 260 | memory |

| | |
|---|---|
| 300 | time |
| 310 | arterial blood pressure |
| 320 | arterial blood pressure waveforms |
| 330 | time instance for which cardiac output is available |
| 340 | time window preceding available cardiac output |

| | |
|---|---|
| 400 | method for training machine learning model |
| 410 | accessing set of training data instances |
| 420 | training on training data instance |
| 430 | selecting input time segment |
| 440 | performing training pass |

| | |
|---|---|
| 500 | method for estimating cardiac output using trained machine learning model |
| 510 | accessing patient monitoring data |
| 520 | providing trained machine learning model |
| 530 | selecting input time segment |
| 540 | obtaining estimated cardiac output |

| | |
|---|---|
| 600 | non-transitory computer-readable medium |
| 610 | data representing computer program |

DETAILED DESCRIPTION OF EMBODIMENTS

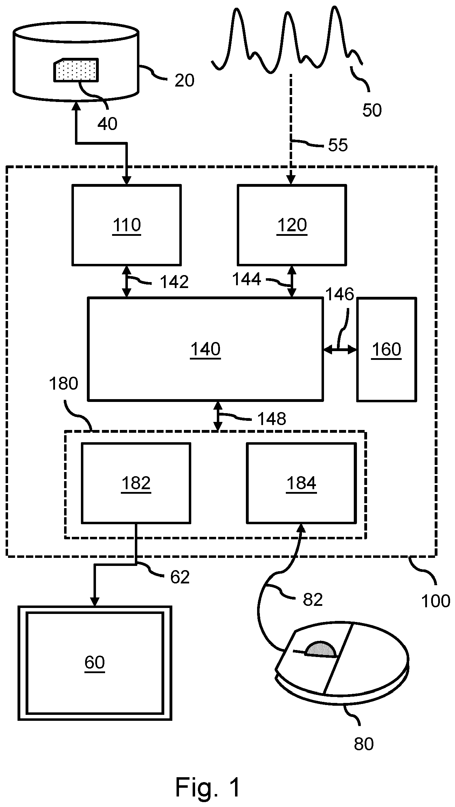

Fig. 1 shows an estimation system 100 which is configured to estimate the cardiac output of a patient from patient monitoring data of the patient. The estimation system 100 is shown to comprise an input interface 120 to access the patient monitoring data 55. The patient monitoring data 55 which is accessed may comprise a time-series of measurements of arterial blood pressure of the patient which may be obtained using an arterial line inserted into an artery of the patient. The time-series of measurements may comprise a sequence of arterial blood pressure waveforms 50, e.g., each comprising a systolic and diastolic phase. The patient monitoring data 55 may in the following also be referred to as 'measurement data' as it contains the measurements of the arterial blood pressure of the patient. The measurement data 55 may be accessed by the estimation system 100 from a patient monitor to which the arterial line is connected. However, this is not a limitation, in that the arterial line may also be directly connected to the estimation system 100. Another example is that the measurement data 55 may be accessed from another entity which is not a patient monitor. In a typical example, the estimation system 100 may receive or otherwise access the measurement data 55 in real-time or near real-time.

The estimation system 100 may further comprise a data storage interface 110 to a data storage 20. The data storage 20 may serve as short term and/or long-term data storage. For example, the measurement data 55 obtained via the input interface 120 may be at least temporarily stored on the data storage 20. In some embodiments, the measurement data 55 may also be accessed from the data storage 20 instead of using another input interface, for example in cases when the measurement data 55 is prerecorded measurement data or when the measurement data 55 represents simulated measurement data. In the example of Fig. 1, the data storage interface 110 is shown to be connected to an external data storage 20. Alternatively, the data storage 20 may be an internal data storage of the estimation system 100 (not shown in Fig. 1). In general, the data storage interface 110 may take various forms, such as a hard disk or solid-state disk interface to one or more hard disks and/or solid state disks, or a network interface to a Local Area Network (LAN).

The estimation system 100 may further comprise a processor subsystem 140 configured to internally communicate with the input interface 120 via data communication 144, with the data storage interface 110 via data communication 142, with a memory 160 via data communication 146 and with a user interface subsystem 180 via data communication 148. The memory 160 may for example be a volatile memory in which a computer program may be loaded which may cause the processor subsystem 140 to carry out functions which are described in this specification as being performed by the estimation system 100.

The user interface subsystem 180 may be an optional component of the estimation system 100, and if present, may be configured to, during operation of the estimation system 100, enable a user to interact with the estimation system 100, for example using a graphical user interface. The user interface subsystem 180 is shown to comprise a user input interface 184 configured to receive user input data 82 from a user input device 80 operable by the user. The user input device 80 may take various forms, including but not limited to a computer mouse, touch screen, keyboard, microphone, etc. Fig. 1 shows the user input device to be a computer mouse 80. In general, the user input interface 184 may be of a type which corresponds to the type of user input device 80, i.e., it may be a thereto corresponding type of user device interface. The user interface subsystem 180 is further shown to comprise a display output interface 182 configured to provide display data 62 to a display 60 to visualize output of the estimation system 100. In the example of Fig. 1, the display is an external display 60. Alternatively, the display may be an internal display.

In some examples, the estimation system 100 may comprise the aforementioned display output interface but not comprise a user input interface. In some examples, the estimation system 100 may comprise another type of output interface, such as an audio interface, e.g., to a loudspeaker, or a network interface. Any of such output interfaces may be used to render or transmit output generated by the estimation system 100.

The processor subsystem 140 may be configured to, during operation of the estimation system 100, provide a trained machine learning model which is trained to estimate cardiac output from a time segment of measurements of the arterial blood pressure, select an input time segment from the patient monitoring data 55, use the input time segment as input to the trained machine learning model to obtain an estimated cardiac output as output, and output the estimated cardiac output via an output interface. The trained machine learning model may for example accessed from the data storage 20, e.g., in form of model data 40 representing the trained machine learning model. As output interface, the display output interface 182 may be used so as to visualize the estimated cardiac output on the display 60. These and other operations of the estimation system 100, and various optional aspects thereof, will be explained in more detail elsewhere in this specification.

In general, the estimation system 100 may be embodied as, or in, a single device or apparatus. The device or apparatus may be a general-purpose device or apparatus, such as a workstation or a computer, but may also be an application-specific device or apparatus, such as a patient monitor. The device or apparatus may comprise one or more microprocessors which may represent the processor subsystem, and which may which execute appropriate software. The software may have been downloaded and/or stored in a corresponding memory, e.g., a volatile memory such as RAM or a non-volatile memory such as Flash. Alternatively, the functional units of the system, e.g., the input interface, the output interface, and the processor subsystem, may be implemented in the device or apparatus in the form of programmable logic, e.g., as a Field-Programmable Gate Array (FPGA). In general, each functional unit of the estimation system 100 may be implemented in the form of a circuit. It is noted that the estimation system 100 may also be implemented in a distributed manner, e.g., involving different devices or apparatuses. For example, the distribution may be in accordance with a client-server model, e.g., using a server and workstation. For example, the user input interface and the display output interface may be part of the workstation, while the processor subsystem may be a subsystem of the server. It is noted that various other distributions are equally conceivable.

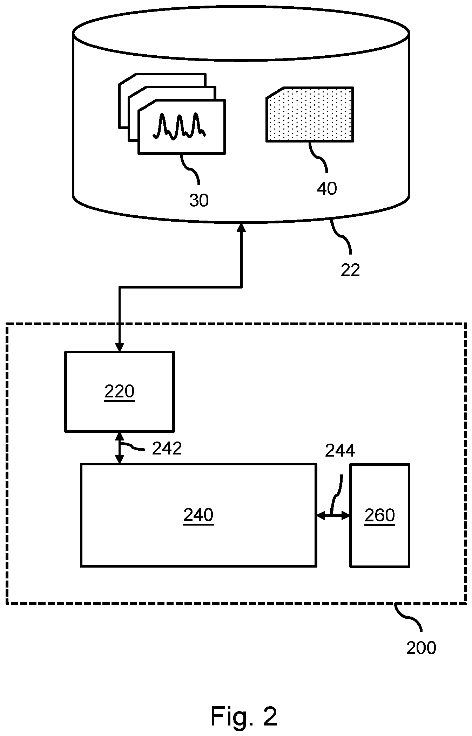

**Fig. 2** shows a training system 200 which is configured to train a machine learning model to estimate cardiac output of a patient from patient monitoring data. The trained machine learning model may then be used by the estimation system Fig. 1 to estimate the cardiac output of patients in clinical practice, as also previously described with reference to Fig.1. The training system 200 is shown to comprise an input interface to access training data to train the machine learning model. In the example of Fig. 2, the input interface is shown to be a data storage interface 220 to an external data storage 22 which comprises the training data 30. The data storage 22 and the data storage interface 220 may for example be of a type as described with reference to Fig. 1 and thereby correspond in type to the data storage 20 and the data storage interface 120 of the estimation system 100. However, this is not a limitation, in that the input interface 220 of the training system 200 may also be of any other type, such as a network interface to a Local Area Network (LAN).

The training data 30 may comprise a set of training data instances. Each or at least a subset of the training data instance may comprise a time-series of measurements of arterial blood pressure of a patient and a quantification of cardiac output of the patient. In other words, the arterial blood pressure measurements of a patient and a quantification of cardiac output of the same patient may together form a training data instance, and several training data instances, e.g., from different patients, may together form the training data 30. The time-series of measurements in each or the subset of training data instances may be obtained by arterial line, for example in a same manner as described with reference to Fig. 1. As such, a time-series may comprise a sequence of arterial blood pressure waveforms.

The training system 200 may further comprise a processor subsystem 240 configured to internally communicate with the input interface 220 via data communication 242 and with a memory 260 via data communication 244. The memory 260 may for example be a volatile memory in which a computer program may be loaded which may cause the processor subsystem 240 to carry out functions which are described in this specification as being performed by the training system. The processor subsystem 240 may be configured to, during operation of the training system 200, train the machine learning model on the training data 30 by, for a respective training data instance, select an input time segment from the time-series of measurements, wherein the input time segment is selected to at least in part temporally precede a time instance for which the quantification of the cardiac output is available, and perform a training pass by using the input time segment as input and the quantification of the cardiac output as prediction target. As a result, a trained machine learning model may be obtained, which may be stored as model data 40, e.g., in the database 22 and which may be transmitted or distributed, e.g., to the aforementioned estimation system. These and other operations of the training system 200, and various optional aspects thereof, will be explained in more detail elsewhere in this specification.

In general, the training system 200 may be embodied as, or in, a single device or apparatus, but also in a distributed manner, e.g., involving different devices or apparatuses, and in a same manner as previously described for the estimation system 100.

With continued reference to the input of the machine learning model, both before and after training, the following is noted. The input to the machine learning model may take the form of measurements data containing a sequence of ABP waveforms which may be measured by A-lines. As is known per se, the two main components of an ABP waveform are the systolic and diastolic phases separated by the dicrotic notch, with the dicrotic notch representing the closure of the aortic valve. Both in the training and in subsequent clinical practice, for the input to the machine learning model to capture adequate information on the cardiac output, ABP waveforms in form of k-minute time segments may be used to estimate the cardiac output at the end of a respective k-minute segment.

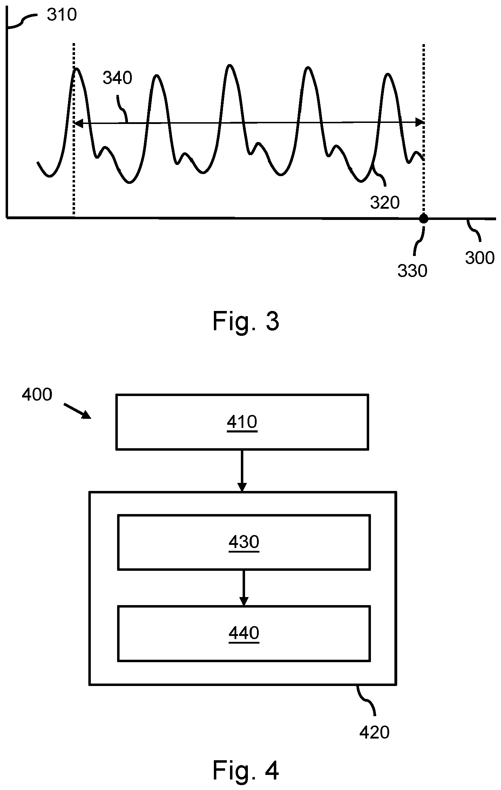

This is illustrated in **Fig. 3**, in which a time-series of arterial blood pressure waveforms 320 is shown in a graph which comprises along the x-axis the time 300 and along the y-axis the arterial blood pressure 310 (e.g., in mmHg). During the training of the machine learning model, a quantification of cardiac output may be available for a particular time instance 330. For example, at time instance 330, the cardiac output may have been measured by thermodilution or using Fick's principle. The arterial blood pressure waveforms 320 within a time-window 340 of k-minutes preceding the time instance 330 may be selected as input to the machine learning model in a training pass while the value of the cardiac output at the time instance 330 may be used as prediction target. For example, the k-minute time window may be anywhere between 30 seconds (e.g., $k=0.5$) and 10 minutes long and may immediately precede the time instance 330 but may also be lie earlier in time than the time instance 330, subject to the time difference between the end of the time window and the time instance 330 preferably not being too large, e.g., being below 5 or 10 minutes . During subsequent clinical use, the trained machine learning model may be used to estimate the cardiac output given k-minutes of the most recent ABP waveforms of the patient. Such estimation may be repeated, e.g., periodically, for example every $n$-th second or $m$-th minute, so as to provide periodic estimates of the cardiac output of the patient.

In clinical practice, the trained machine learning model may be executed by a patient monitor which is connected to the patient's A-line. In such examples, the patient monitor may implement or comprise the aforementioned estimation system. As it is desirable for the cardiac output to be estimated in real-time or near-real time, and considering that patient monitors and similar devices may be resource constrained, the machine learning model may be trained to be applied, and subsequently applied, to the raw arterial blood pressure waveforms without further feature extraction or segmentation. As such, a *k*-minute time window of ABP waveforms may be used as input to the trained machine learning model with only basic or no pre-processing. Examples of such basic pre-processing include replacing ABP data that is outside a plausibility range with "NaN" values and scaling the ABP waveforms to a predetermined range, e.g., between 0 and 1 or between -1 and 1.

With continued reference to the training of the machine learning model, it is noted that the training may be considered as a supervised machine learning problem in which labelled examples are used to train the machine learning model. In experiments, the MIMIC III matched waveform database *(Moody, B., Moody, G., Villarroel, M., Clifford, G. D., & Silva, I. (2020). MIMIC-III Waveform Database Matched Subset (version 1.0). PhysioNet. https://doi.org/10.13026/c2294b)* was used as a source for training data since the MIMIC III database contains both measurements of cardiac output and ABP waveforms. For example, cardiac output may be available as several items in the MIMIC III database:

| Item identifier | Label |
|---|---|
| 89 | C.O. (fick) |
| 90 | C.O. (thermodilution) |
| 8048 | C.O. |
| 2372 | FICK CO |
| 41946 | CARDIAC OUTPUT |
| 228369 | Cardiac Output (CO NICOM) |
| 227543 | CO (Arterial) |
| 220088 | Cardiac Output (thermodilution) |
| 228178 | CO (PiCCO) |

From the MIMIC III database, ABP waveforms may be selected as input for the training for those patients for which the database also contains a cardiac output measurement. In a specific examples, only those patients may be included in the training data for whom ABP waveforms of one minute or longer are available inside a ten-minute window immediately prior to each cardiac output measurement. It is noted that a patient may have multiple pairs of cardiac output measurements and ABP waveforms and thus contribute to multiple labelled examples, e.g., provide multiple training data instances. In specific experiments which were performed using the MIMIC III database, the training data consisted of 12,708 paired CO-ABP waveform segments from 663 unique patients. It is noted that in other examples, only ABP waveforms may be selected for patients for which a particular type of cardiac output measurement is available, for example cardiac output obtained by thermodilution (e.g., item ID 90 and 220088 in the MIMIC III database).

As machine learning model, a temporal convolutional network (TCN) may be used, as described in *"Bai, S., Kolter, J. Z., & Koltun, V. (2018). An empirical evaluation of generic convolutional and recurrent networks for sequence modeling. arXiv preprint arXiv:1803. 01271 "*. A typical example of a TCN comprises several stacked layers. Each layer may comprise a series of temporal convolutional blocks. Each temporal convolutional block may comprise a dilated convolutional layer followed by an optional activation function such as ReLU (Rectified Linear Unit) or tanh. The dilated convolutional layer may help to capture long-range dependencies by incorporating different dilation rates. In each layer, the temporal convolutional blocks are typically followed by a downsampling operation, such as pooling or strided convolutions, to reduce the temporal resolution. This downsampling helps in reducing the computational complexity and capturing higher-level temporal patterns. The output of the last layer is usually flattened and fed into fully connected layers or other classification/regression modules for final prediction or estimation. Moreover, in the training of the TCN, drop-out may be used as a form of regularization to prevent overfitting.

In a TCN model, the number of layers, filter sizes, dilation rates, and other architectural parameters may vary based on the specific implementation and task requirements. In experiments using trained data obtained from the MIMIC III database, the estimation of the cardiac output was setup as a regression problem. The labelled dataset was partitioned into train and test partitions such that a patient is represented in either one of the partitions. The TCN model was trained on 80% of the patients' data and the remaining 20% as used as test data. A TCN model was trained to estimate the cardiac output using the following settings: number of hidden units per layer=10, kernel size=375 samples (125 samples/second x 3 seconds), dropout=0.05, number of epochs=100 and batch size=32. The mean absolute error (MAE) was used as a loss function since it is less sensitive to outliers. The hyperparameters were tuned on the train dataset using the MAE as a metric. The resulting trained TCN provides an estimate of the cardiac output given an ABP segment of length k minutes. The cardiac output estimate may be obtained from the model as:

$$Y = W^T X + b$$

where Y is the estimated cardiac output, W are the matrix of TCN weights, X is ABP waveform segment and b is a vector of bias terms.

While the above refers to the use of a TCN model as a preferred example of a machine learning model, other types of machine learning model may be used instead of a TCN model, provided that the machine learning model is capable of capturing local dependencies to capture a shape of individual ABP waveforms and capturing long-term dependencies in the sequence of ABP waveforms. For example, so-called Long Short-Term Memory (LSTM) models or Gated Recurrent Unit (GRU) models have shown at least some proficiency in handling both short-term and long-term dependencies in sequence data.

**Fig. 4** shows a method 400 for training the machine learning model. The method 400 may correspond to an operation of the training system 200 of Fig. 2. However, this is not a limitation, in that the computer-implemented method 400 may also be performed using another system, apparatus or device. The method 400 may comprise, in a step titled "ACCESSING SET OF TRAINING DATA INSTANCES", accessing 410 training data, wherein the training data comprises a set of training data instances as described elsewhere in this specification, in a step titled "TRAINING ON TRAINING DATA INSTANCE", training 420 the machine learning model on the training data by, for a respective training data instance, in a step titled "SELECTING INPUT TIME SEGMENT", selecting 430 an input time segment from the time-series of measurements, wherein the input time segment is selected to at least in part temporally precede a time instance for which the quantification of the cardiac output is available, and in a step titled "PERFORMING TRAINING PASS", performing 440 a training pass by using the input time segment as input and the quantification of the cardiac output as prediction target.

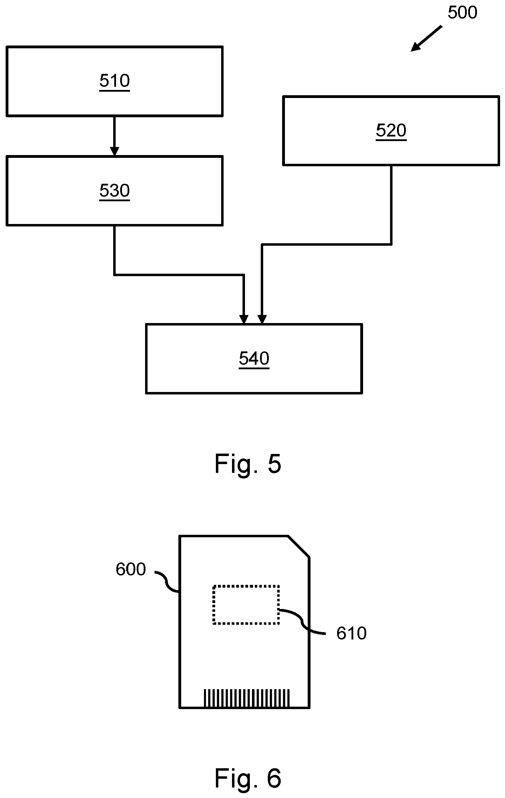

**Fig. 5** shows a method 500 for using the trained machine learning model to estimate the cardiac output of a patient from arterial blood pressure measurement data. The method 500 may correspond to an operation of the estimation system 100 of Fig. 1. However, this is not a limitation, in that the computer-implemented method 500 may also be performed using another system, apparatus or device. The method 500 may comprise, in a step titled "ACCESSING PATIENT MONITORING DATA", accessing 510 patient monitoring data as described elsewhere in this specification, e.g., in form of arterial blood pressure measurement data, in a step titled "PROVIDING TRAINED MACHINE LEARNING MODEL", providing 520 a trained machine learning model which is trained to estimate cardiac output from a time segment of measurements of the arterial blood pressure, in a step titled "SELECTING INPUT TIME SEGMENT", selecting 530 an input time segment from the patient monitoring data, and in a step titled "OBTAINING ESTIMATED CARDIAC OUTPUT", using the input time segment as input to the trained machine learning model to obtain 540 an estimated cardiac output as output.

It will be appreciated that steps of the method 400 of Fig. 4 and the method 500 of Fig. 5 may be performed in any suitable order, e.g., consecutively, simultaneously, or a combination thereof, subject to, where applicable, a particular order being necessitated, e.g., by input/output relations. Steps may also be performed as part of other steps.

Any method described in this specification may be implemented on a computer as a computer-implemented method, as hardware, or as a combination of both. As also illustrated in **Fig. 6,** instructions for the computer, e.g., executable code, may be stored on a computer readable medium 600, e.g., in the form of a series 610 of machine-readable physical marks and/or as a series of elements having different electrical, e.g., magnetic, or optical properties or values. The executable code may be stored in a transitory or non-transitory manner. Examples of computer readable mediums include memory devices, optical storage devices, integrated circuits, etc. Fig. 6 shows a memory device 600.

The following provides a non-limiting summary of some aspects of the invention: a system and method may be provided for estimating cardiac output of a patient from patient monitoring data. The patient monitoring data may comprise a sequence of arterial blood pressure waveforms. A trained machine learning model may be provided which may have been previously trained to estimate the cardiac output from a sequence of arterial blood pressure waveforms. The trained machine learning model may be used for this purpose by selecting an input time segment from the patient monitoring data and using the input time segment as input to the trained machine learning model to obtain an estimated cardiac output as output. Advantageously, by training and applying a machine learning model directly on arterial blood pressure waveforms, instead of on features extracted from the arterial blood pressure waveforms, it may be avoided that inaccuracies and errors in the feature extraction, and in the segmentation of the waveform which typically precedes such feature extraction, propagate into the estimate of the cardiac output.

Examples, embodiments or optional features, whether indicated as non-limiting or not, are not to be understood as limiting the invention as claimed.

It should be noted that the above-mentioned embodiments illustrate rather than limit the invention, and that those skilled in the art will be able to design many alternative embodiments without departing from the scope of the appended claims. In the claims, any reference signs placed between parentheses shall not be construed as limiting the claim. Use of the verb "comprise" and its conjugations does not exclude the presence of elements or stages other than those stated in a claim. The article "a" or "an" preceding an element does not exclude the presence of a plurality of such elements. Expressions such as "at least one of" when preceding a list or group of elements represent a selection of all or of any subset of elements from the list or group. For example, the expression, "at least one of A, B, and C" should be understood as including only A, only B, only C, both A and B, both A and C, both B and C, or all of A, B, and C. The invention may be implemented by means of hardware comprising several distinct elements, and by means of a suitably programmed computer. In the device claim enumerating several means, several of these means may be embodied by one and the same item of hardware. The mere fact that certain measures are recited in mutually different dependent claims does not indicate that a combination of these measures cannot be used to advantage.

**Claims**

1.  A training system (200) to train a machine learning model to estimate cardiac output of a patient from patient monitoring data, wherein the training system comprises:

    - an input interface (220) to access training data (30), wherein the training data comprises a set of training data instances, wherein each training data instance comprises a time-series of measurements of arterial blood pressure of a patient and a quantification of cardiac output of the patient, wherein the time-series of measurements is obtained by arterial line and comprises a sequence of arterial blood pressure waveforms (320);
    - a processor subsystem (240) configured to train the machine learning model (40) on the training data by, for a respective training data instance:
    - select an input time segment (340) from the time-series of measurements, wherein the input time segment is selected to at least in part temporally precede a time instance (330) for which the quantification of the cardiac output is available;
    - perform a training pass by using the input time segment as input and the quantification of the cardiac output as prediction target.

2.  The system (200) according to claim 1, wherein the machine learning model (40) is or comprises a temporal convolutional network.

3.  The system (200) according to claim 1 or 2, wherein the processor subsystem (240) is configured to select the input time segment (340) to have a time length between 30 seconds and 10 minutes.

4.  The system (200) according to any one of claims 1 to 3, wherein the processor subsystem (240) is configured to select the input time segment (340) within or as a time window which ends at or is temporally adjacent to the time instance (330) for which the quantification of the cardiac output is available, for example within a ten-minute time window.

5.  The system (200) according to any one of claims 1 to 4, wherein the processor subsystem (240) is configured to use or exclusively use quantifications of cardiac output which are obtained by measurement using thermodilution.

6.  The system (200) according to any one of claims 1 to 5, wherein the processor subsystem (240) is configured to exclusively use the input time segment (340) as input to the machine learning model.

7.  The system (200) according to any one of claims 1 to 6, wherein the processor subsystem (240) is configured to normalize measurements within the input time segment before using the input time segment in the training pass to fit within a predetermined range, for example between 0 and 1 or between -1 and 1.

8.  An estimation system (100) to estimate cardiac output of a patient from patient monitoring data, wherein the estimation system comprises:

    - an input interface (120) to access the patient monitoring data (55), wherein the patient monitoring data comprises a time-series of measurements of arterial blood pressure of the patient, wherein the time-series of measurements is obtained by arterial line and comprises a sequence of arterial blood pressure waveforms (50, 320);

- an output interface (182);
- a processor subsystem (140) configured to:

- provide a trained machine learning model which is trained to estimate cardiac output from a time segment of measurements of the arterial blood pressure;
- select an input time segment (340) from the patient monitoring data;
- use the input time segment as input to the trained machine learning model to obtain an estimated cardiac output as output; and
- output the estimated cardiac output via the output interface.

9. The system (100) according to claim 8, wherein the input interface (120) is configured to access the patient monitoring data as a real-time or near-real time stream of measurements, and wherein the processor subsystem (140) is configured to provide periodic estimates of the cardiac output by periodically selecting an input time segment which includes and extends into the past from a most recent measurement.

10. The system (100) according to claim 9, wherein a periodicity of the estimates of the cardiac output and a length of the input time segment are selected such that consecutive input time segments overlap in time.

11. The system (100) according to any one of claims 8 to 10, wherein the processor subsystem (140) is configured to select the input time segment (340) to have a time length between 30 seconds and 10 minutes.

12. A patient monitor comprising the system (100) according to any one of claims 1 to 11.

13. A computer-implemented method (400) for training a machine learning model to estimate cardiac output of a patient from patient monitoring data, wherein the method comprises:

- accessing (410) training data, wherein the training data comprises a set of training data instances, wherein each training data instance comprises a time-series of measurements of arterial blood pressure of a patient and a quantification of cardiac output of the patient, wherein the time-series of measurements is obtained by arterial line and comprises a sequence of arterial blood pressure waveforms;
- training (420) the machine learning model on the training data by, for a respective training data instance:
- selecting (430) an input time segment from the time-series of measurements, wherein the input time segment is selected to at least in part temporally precede a time instance for which the quantification of the cardiac output is available;
- performing (440) a training pass by using the input time segment as input and the quantification of the cardiac output as prediction target.

14. A computer-implemented method (500) for estimating cardiac output of a patient from patient monitoring data, wherein the method comprises:

- accessing (510) the patient monitoring data, wherein the patient monitoring data comprises a time-series of measurements of arterial blood pressure of the patient, wherein the time-series of measurements is obtained by arterial line and comprises a sequence of arterial blood pressure waveforms;
- providing (520) a trained machine learning model which is trained to estimate cardiac output from a time segment of measurements of the arterial blood pressure;
- selecting (530) an input time segment from the patient monitoring data; and
- using the input time segment as input to the trained machine learning model to obtain (540) an estimated cardiac output as output.

15. A transitory or non-transitory computer-readable medium (600) comprising data (610) representing a computer program, the computer program comprising instructions for causing a processor system to perform the method according to claim 13 or 14.

Fig. 1

Fig. 2

310

340

320

330    300

Fig. 3

400

410

430

440

420

Fig. 4

Fig. 5

Fig. 6

Europäisches
Patentamt

European
Patent Office

Office européen
des brevets

**EUROPEAN SEARCH REPORT**

Application Number

EP 23 18 5449

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | EP 4 111 955 A2 (SEOUL NAT UNIV HOSPITAL [KR]; DAEGU GYEONGBUK INST SCIENCE & TECH [KR]) 4 January 2023 (2023-01-04) * the whole document * | 1-15 | INV. A61B5/021 A61B5/029 A61B5/00 G16H50/20 |
| X | CN 113 057 617 A (UNIV CHONGQING TECHNOLOGY) 2 July 2021 (2021-07-02) * the whole document * | 1-15 | ADD. G06N3/08 |
| X | CN 111 493 855 A (UNIV CHONGQING TECHNOLOGY) 7 August 2020 (2020-08-07) * the whole document * | 1-15 | |
| X | US 2021/282724 A1 (NATARAJAN ANNAMALAI [US] ET AL) 16 September 2021 (2021-09-16) * paragraphs [0004] - [0007] * * paragraphs [0050] - [0056] * | 1-15 | |

TECHNICAL FIELDS SEARCHED (IPC)

A61B
G06N
G16H

The present search report has been drawn up for all claims

| Place of search | Date of completion of the search | Examiner |
|---|---|---|
| The Hague | 27 November 2023 | Loveniers, Kris |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons

& : member of the same patent family, corresponding
   document

EPO FORM 1503 03.82 (P04C01)

**ANNEX TO THE EUROPEAN SEARCH REPORT
ON EUROPEAN PATENT APPLICATION NO.**                    EP 23 18 5449

This annex lists the patent family members relating to the patent documents cited in  the above-mentioned European search report.
The members are as contained in the European Patent Office EDP file on
The European Patent Office is in no way liable for these particulars which are merely given for the purpose of information.

27-11-2023

| Patent document cited in search report | | Publication date | Patent family member(s) | | Publication date |
|---|---|---|---|---|---|
| EP 4111955 | A2 | 04-01-2023 | EP | 4111955 A2 | 04-01-2023 |
| | | | KR | 20210108105 A | 02-09-2021 |
| | | | US | 2023355185 A1 | 09-11-2023 |
| | | | WO | 2021172852 A2 | 02-09-2021 |
| CN 113057617 | A | 02-07-2021 | NONE | | |
| CN 111493855 | A | 07-08-2020 | NONE | | |
| US 2021282724 | A1 | 16-09-2021 | CN | 115279260 A | 01-11-2022 |
| | | | EP | 4117514 A1 | 18-01-2023 |
| | | | JP | 2023517567 A | 26-04-2023 |
| | | | US | 2021282724 A1 | 16-09-2021 |
| | | | WO | 2021180500 A1 | 16-09-2021 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 113057617 A

**Non-patent literature cited in the description**

- Estimating cardiac output from arterial blood pressure waveforms: a critical evaluation using the MIMIC II database. **SUN, J. X.** ; **REISNER, A. T.** ; **SAEED, M.** ; **MARK, R. G.** Computers in Cardiology. IEEE., September 2005, vol. 2005, 295-298

- **BAI, S.** ; **KOLTER, J. Z.** ; **KOLTUN, V.** An empirical evaluation of generic convolutional and recurrent networks for sequence modeling. *arXiv:1803. 01271*, 2018